# EUROPEAN PATENT APPLICATION

(11) **EP 2 386 301 A1**
(43) Date of publication of application: **16.11.2011**
(21) Application number: 10809123.2
(22) Date of filing: 22.06.2010
(51) Int. Cl.: A61K 31/4422, A61K 31/353, A61K 31/138, A61K 31/17, A61P 9/00, A61P 9/12, A61P 9/04

(54) **PHARMACEUTICAL COMPOSITION OF LEVOAMLODIPINE OR PHARMACEUTICALLY ACCEPTABLE SALTS THEREOF AND BLOCKERS, AND USE THEREOF**

(30) Priority: 09.02.2010 CN 201010107915
(71) Applicant: Shihuida Pharmaceuticals Group (Jilin) Ltd, Shanghai 200439 (CN)
(72) Inventor: YANG, Ling, Shanghai 200439 (CN); XUE, Chuan Xiao, Shanghai 200439 (CN); ZHANG, Xi Tian, Shanghai 200439 (CN); LI, Huan, Shanghai 200439 (CN); SONG, Sen Tao, Shanghai 200439 (CN)
(74) Representative: Herrmann, Uwe
(86) International application number: PCT/CN2010/074190
(87) International publication number: WO 2011/097860

(57) **Abstract**

A pharmaceutical composition of levamlodipine or a pharmaceutically acceptable salt thereof and a β receptor blocking agent, and a use thereof are provided. An active ingredient of the pharmaceutical composition contains levamlodipine or a pharmaceutically acceptable salt thereof, and a β receptor blocking agent, in which the β receptor blocking agent is one selected from nebivolol, bisoprolol, betaxolol, celiprolol, and nadolol. A use of the pharmaceutical composition in preparation of a medicine for treating hypertension is further provided. The pharmaceutical composition of the prevent invention has the advantages that the therapeutic effect is obvious, and the administration is convenient.

## Description

### BACKGROUND OF THE INVENTION

### Field of Invention

The present invention relates to a medicine for treating hypertension, and more particularly to a composition containing levamlodipine or a pharmaceutically acceptable salt thereof, and a β receptor blocking agent, and a use thereof.

### Related Art

In recent years, with the continuous improvement of living standards and the changes in the dietary structure of Chinese people, the increase of life stress, and the increase of the elderly population, the incidence of hypertension is gradually increased, and at the same time, hypertension causes lesions to heart, brain, kidneys, and other organs, is closely related to sugar and lipid metabolism disorders and diabetes, significantly reduces the quality of life of patients, and even threatens the lives of patients in serious cases. According to the global mortality statistics of various diseases of the World Health Organization (WHO), the cardiovascular disease deaths represented by hypertension accounts for 36% of the total number of deaths; therefore, improving people's awareness on hypertension is of great importance for early-stage prevention and timely treatment.

Levamlodipine is an optically pure medicine that is firstly chirally resolved in China, an anti-hypertensive medicine that is firstly chirally resolved in the world, and a long-acting basic dihydropyridine calcium antagonist. Levamlodipine functions through a site (N site) attached to dihydropyridine on a cell, and blocks calcium ions from entering the cardiac and vascular smooth muscle cells in a transmembrane manner, so as to relax the smooth muscle, decrease the vascular resistance, and lower the blood pressure. Presently, clinical trial evidences indicate that, therapeutic dose of levamlodipine has very slight or no influence on cardiac contractility and atrioventricular conduction, and levamlodipine is a medicine with the minimal effect on sympathetic excitation among the calcium antagonists. Levamlodipine may also be used to treat hypertension associated with heart failure, reverse ventricular hypertrophy, improve the relaxation function of the heart during the diastolic stage, protect the renal function with mild diuretic function, and prevent coronary heart disease, myocardial infarction, and stroke, and may further partially reverse abnormal circadian rhythm of blood pressure, and have mild anti-platelet effect, anti-myocardial ischemia effect, anti-arrhythmia effect, insulin sensitivity increasing effect and a certain anti-atherosclerosis effect. However, levamlodipine may induce secondary rising of epinephrine and norepinephrine, increase heart rate, and have adverse reaction such as headache and flush, so a considerable of patients withdraw, and the clinical application of levamlodipine is limited.

β receptor blocking agents are a type of medicines capable of selectively binding to a β-adrenergic receptor, to antagonize the agonizing effects of neurotransmitter and catecholamine on β receptor. Clinical studies indicate that the β receptor blocking agent is suitable for treating different levels of hypertension, and is also suitable for treating patients having hypertension associated with angina, myocardial infarction, fast arrhythmia, congestive heat failure, and pregnancy. The β receptor blocking agent has strong effects of decreasing myocardial oxygen consumption, antagonizing arrhythmia caused by catecholamine, improving ventricular fibrillation threshold, anti-platelet, decreasing cardiovascular impairment, lowering myocardial re-infarction rate, and improving left ventricular remodeling after infarction. Lots of experiments with β receptor blocking agent (CIBIS II, MERIT-HF, and COPERNICUS) indicate that use of the β receptor blocking agent in long time can decrease the total mortality of heart failure patients, the mortality of cardiovascular diseases, the cardiogenic sudden death, and the death caused by deterioration of heart failure. The β receptor blocking agent is frequently used for treatment of fast arrhythmia, including sinus tachycardia, premature atrial contraction, ventricular extrasystoles, auricular tachycardia, supraventricular tachycardia, and ventricular tachycardia. As for patients having the symptom of hypertrophic cardoiomyopathy, the β receptor blocking agent is a preferred therapy, can control the ventricular rate, decrease the cardiac contractility, maximize the ventricular filling and end-diastolic volume, and modify the myocardial compliance, and may be used for treatment of, for example, dilated cardiomyopathy with or without heart failure, or alleviate symptoms, prevent sudden death, and improve prognosis. The common adverse effects caused by the β receptor blocking agent include, for example, orthostatic hypotension and bronchospasm.

The common anti-hypertensive medicine further includes various other agents such as diuretic anti-hypertensive agents, central nerve system and sympathetic nerve depressors, enzyme inhibitors, and vasodilators with different adverse effects. Although combined administration of anti-hypertensive medicines are reported, because such a combined administration can lower the dosage of a single medicine, thus decreasing the adverse effects, and the combined administration of some medicines may also counteract the adverse effects; however, the combined administration of many medicines may not generate a synergistic anti-hypertensive effect, and in this case, the contribution to lowering of the dosage of the medicine is limited. In order to further lower the adverse effects and dosage of the antihypertensive medicines, it is necessary to find medicines capable of generating the synergistic effect.

### SUMMARY OF THE INVENTION

The present invention is directed to a novel pharmaceutical composition for treating hypertension which has a synergistic effect, so as to achieve a therapeutic effect of medicines administrated in combination for treating medium to severe hypertension superior to that of a medicine administrated alone.

In the pharmaceutical composition for treating hypertension according to the present invention, the active ingredient includes levamlodipine or a pharmaceutically acceptable salt thereof, and a β receptor blocking agent, in which the β receptor blocking agent is preferably one of nebivolol, bisoprolol, betaxolol, celiprolol, or nadolol.

The pharmaceutically acceptable salt of levamlodipine is selected from levamlodipine benzenesulfonate, levamlodipine mesylate, levamlodipine acetate, levamlodipine aspartate, levamlodipine tartrate, levamlodipine maleate, levamlodipine sulfate, levamlodipine hydrochloride, or levamlodipine hydrobromide, and preferably levamlodipine benzenesulfonate. Levamlodipine may be prepared through many methods, for example, the methods described in CN1100038C and CN100364976C, and based on which, a stable levamlodipine salt may be prepared by a routine neutralization reaction between an acid and a base.

The molecular formulas of the above medicines are as follows:

levamlodipine: 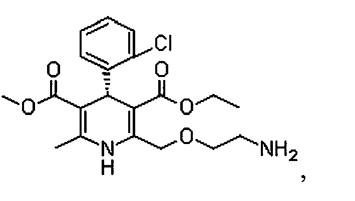 levamlodipine benzenesulfonate: 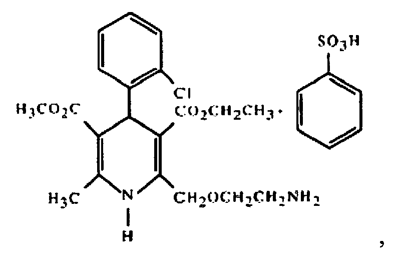 nebivolol: 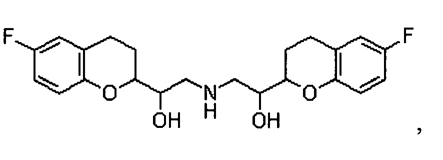 bisoprolol: 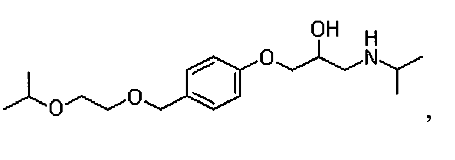 betaxolol: 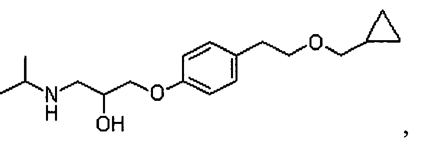 celiprolol: 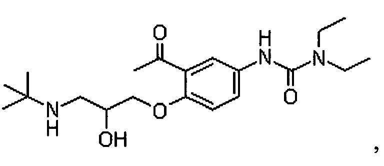 and nadolol:

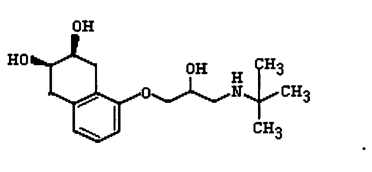

In the pharmaceutical composition, a mixing ratio of levamlodipine or pharmaceutically acceptable salt thereof based on levamlodipine to nebivolol is 1:0.2-2, and preferably 1:0.5-1, by weight; a mixing ratio of levamlodipine or pharmaceutically acceptable salt thereof based on levamlodipine to bisoprolol is 1:0.2-4, and preferably 1:0.5-1, by weight; a mixing ratio of levamlodipine or pharmaceutically acceptable salt thereof based on levamlodipine to betaxolol is 1:1-8, and preferably 1:2-4, by weight; a mixing ratio of levamlodipine or pharmaceutically acceptable salt thereof based on levamlodipine to celiprolol is 1:10-120, and preferably 1:20-40, by weight; and a mixing ratio of levamlodipine or pharmaceutically acceptable salt thereof based on levamlodipine to nadolol is 1:4-128, and preferably 1:8-16, by weight.

The preferred content of the active ingredient in per unit preparation of the pharmaceutical composition is: the content of levamlodipine or the pharmaceutically acceptable salt thereof based on levamlodipine is 1.0-30 mg, and preferably 2.5-5 mg; the content of nebivolol is 0.5-10 mg, and preferably 1.25-5 mg; the content of bisoprolol is 0.5-20 mg, and preferably 1.25-10 mg; the content of betaxolol is 2.5-40 mg, and preferably 5-20 mg; the content of celiprolol is 25-600 mg, and preferably 50-300 mg; and the content of nadolol is 10-640 mg, and preferably 20-320 mg.

The pharmaceutical composition contains a suitable amount of a pharmaceutically acceptable adjuvant, which is selected form microcrystalline cellulose, pregelatinized starch, lactose, carboxymethyl starch sodium, magnesium stearate, and talc. Moreover, suitable amount of other pharmaceutical acceptable diluent, adhesive, disintegrant, lubricant, flavoring agent, and flavoring agent may be further added.

The pharmaceutical composition may be prepared into an oral preparation, including a tablet or a capsule, in which the tablet may be coated with a sugar coat or a film, or be uncoated.

The present invention further provides a use of the pharmaceutical composition in preparation of a medicine for preventing hypertension.

In the present invention, a compound preparation of levamlodipine and a designated β receptor blocking agent not only has a synergistic anti-hypertensive effect, but also exerts the advantages of the two anti-hypertensive medicines, and the single dosages of levamlodipine and the β receptor blocking agent in the compound preparation are reduced, such that the adverse effects caused by a high-dosage single medicine are lowered, the patient compliance is good, and the incidence of cardio-cardiovascular events is decreased, thus improving the quality of life of the patient. The compound preparation of the present invention is useful for using in senile patients having hypertension associated with heart failure or having hypertension associated with atrial fibrillation.

### BRIEF DESCRIPTION OF THE DRAWINGS

No drawings.

### DETAILED DESCRIPTION OF THE INVENTION

The present invention is further described with the following embodiments, in which Embodiments 1-15 are implementations of the preparations of levamlodipine and a β receptor blocking agent, or pharmaceutically acceptable salts thereof (hereinafter, the weights are calculated based on levamlodipine, nebivolol, bisoprolol, betaxolol, celiprolol, and nadolol), and Embodiment 16 is an implementation of a pharmacological experiment; however, the application scope of the present invention is not limited to the embodiments.

### Embodiments 1-10: Preparation of compound tablets

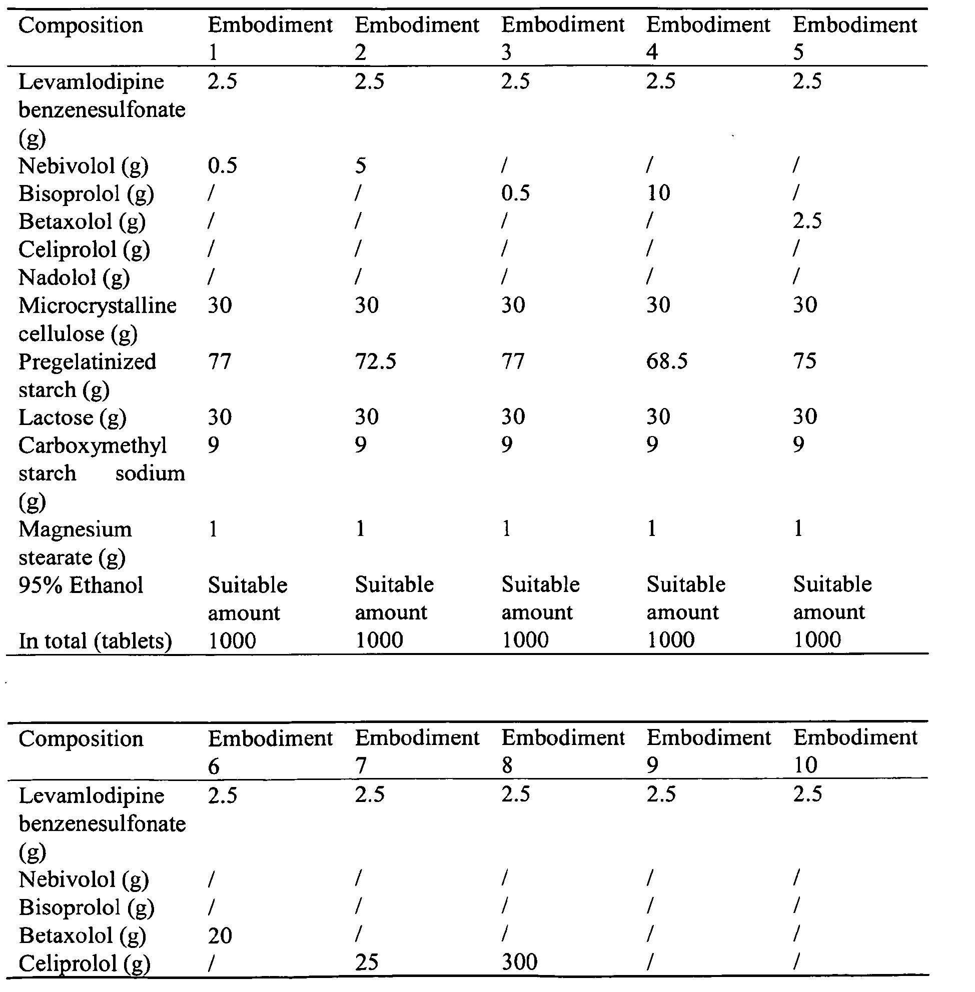

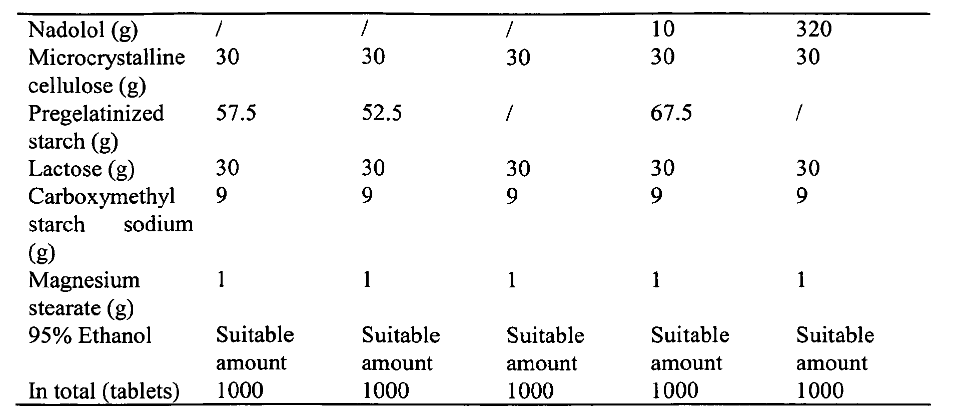

Preparation process: levamlodipine, a β receptor blocking agent, microcrystalline cellulose, pregelatinized starch, lactose, carboxymethyl starch sodium were placed in a mortar, uniformly mixed by grinding, screened with a 20-mesh sieve, added into a suitable amount of 95% ethanol to prepare a soft material, screened with a 20-mesh sieve, granulated, and air dried at 40°C. The dried granule was finished with a 16-mesh sieve, added with magnesium stearate, uniformly mixed, and then tableted.

Administration method: orally administrating 1-2 tablets at morning, once daily.

### Embodiments 11-15: Preparation of compound capsules

| Composition | Embodiment 11 | Embodiment 12 | Embodiment 13 | Embodiment 14 | Embodiment 15 |
|---|---|---|---|---|---|
| Levamlodipine benzenesulfonate (g) | 2.5 | 2.5 | 2.5 | 2.5 | 2.5 |
| Nebivolol (g) | 1.25 | / | / | / | / |
| Bisoprolol (g) | / | 1.25 | / | / | / |
| Betaxolol (g) | / | / | 5 | / | / |
| Celiprolol (g) | / | / | / | 50 | / |
| Nadolol (g) | / | / | / | / | 20 |
| Microcrystalline cellulose (g) | 134 | 135 | 131 | 86 | 116 |
| Talc (g) | 4 | 4 | 4 | 4 | 4 |
| 0.5% PVP-k30 ethanol solution | Suitable amount | Suitable amount | Suitable amount | Suitable amount | Suitable amount |
| In (capsules) | total 1000 | 1000 | 1000 | 1000 | 1000 |

Preparation process: levamlodipine, a β receptor blocking agent, and microcrystalline cellulose were placed in a mortar, uniformly mixed by grinding, screened with a 20-mesh sieve, added suitable amount of 0.5% PVP-k30 ethanol solution prepare a soft material, screened with a 20-mesh sieve, granulated, and air dried at 40°C. The dried granule was finished with a 16-mesh sieve, added with talc, uniformly mixed, and then capsulated.

Administration method: orally administrating 1-2 capsules at morning, once daily.

Other salts of levamlodipine may be used in place of the component levamlodipine benzenesulfonate, and formulated into a capsule or a tablet with the rest other ingredients with reference to the above preparation processes, and persons of skill in the art may also prepare other formulations falling in the protection scope of the present invention by altering the amounts of corresponding components.

### Emobodiment16: Antihypertension test in rat

Experiment method: 120 SHR rats having spontaneous hypertension (female:male 1:2, weighted 200-240 g) were equally divided into 12 groups according to the level of hypertension (the specific grouping method is as shown in Table 1), and intragastrically administrated with the medicine. For the blood pressure of the rat, the systolic pressure at the tail artery of the rat when being wake and quiet was indirectly measured by an electronic blood pressure meter through tail volume method respectively before administration, and at the end of 1, 2, 3, and 4 weeks after administration.

**Table 1: Grouping method**

| Model group | Equal volume of 0.9% saline | / |
|---|---|---|
| Single medicine | Levamlodipine benzenesulfonate | 5 mg·kg⁻¹·d⁻¹ |
| group 1 Single | Nebivolol | 5 mg·kg⁻¹·d⁻¹ |
| medicine group 2 | | |
| Single medicine group 3 | Bisoprolol | 5 mg·kg⁻¹·d⁻¹ |
| Single medicine group 4 | Betaxolol | 10 mg·kg⁻¹·d⁻¹ |
| Single medicine group 5 | Celiprolol | 100 mg·kg⁻¹·d⁻¹ |
| Single medicine group 6 | Nadolol | 40 mg·kg⁻¹·d⁻¹ |
| Compound group 1 | Levamlodipine benzenesulfonate/nebivolol | 2.5 mg·kg⁻¹·d⁻¹/1.25mg·kg⁻¹·d⁻¹ |
| Compound group 2 | Levamlodipine benzenesulfonate/nebivolol | 2.5 mg·kg⁻¹·d⁻¹/2.5mg·kg⁻¹·d⁻¹ |
| Compound group 3 | Levamlodipine benzenesulfonate/bisoprolol | 2.5 mg·kg⁻¹·d⁻¹/1.25mg-kg⁻¹·d⁻¹ |
| Compound group 4 | Levamlodipine benzenesulfonate/bisoprolol | 2.5 mg·kg⁻¹·d⁻¹/2.5mg-kg⁻¹·d⁻¹ |
| Compound group 5 | Levamlodipine benzenesulfonate/betaxolol | 2.5 mg·kg⁻¹·d⁻¹/5mg·kg⁻¹·d⁻¹ |
| Compound group 6 | Levamlodipine benzenesulfonate/betaxolol | 2.5 mg.kg⁻¹·d⁻¹/10mg·kg⁻¹·d⁻¹ |
| Compound group 7 | Levamlodipine benzenesulfonate/celiprolol | 2.5 mg.kg⁻¹·d⁻¹/50mg·kg⁻¹·d⁻¹ |
| Compound group 8 | Levamlodipine benzenesulfonate/celiprolol | 2.5 mg.kg⁻¹·d⁻¹/100mg·kg⁻¹·d⁻¹ |
| Compound group 9 | Levamlodipine benzenesulfonate/nadolol | 2.5 mg·kg⁻¹·d⁻¹/20mg·kg⁻¹·d⁻¹ |
| Compound group 10 | Levamlodipine benzenesulfonate/nadolol | 2.5 mg·kg⁻¹·d⁻¹/40mg·kg⁻¹·d⁻¹ |

Experimental results: compared with the model group, the single medicine group 1, the single medicine group 2, the single medicine group 3, the single medicine group 4,v single medicine group 5, the single medicine group 6, the compound group 1, the compound group 2, the compound group 3, the compound group 4, the compound group 5, the compound group 6, the compound group 7, the compound group 8, the compound group 9, and the compound group 10 have significant anti-hypertensive effect; the compound group 1 and the compound group 2 have significant anti-hypertensive effect compared with the single medicine group 1 and single medicine group 2; the compound group 3 and the compound group 4 have significant anti-hypertensive effect compared with the single medicine group 1 and the single medicine group 3; the compound group 5 and the compound group 6 have significant anti-hypertensive effect compared with the single medicine group 1 and the single medicine group 4; the compound group 7 and the compound group 8 have significant anti-hypertensive effect compared with the single medicine group 1 and single medicine group 5; the compound group 9 and compound group 10 have significant anti-hypertensive effect compared with the single medicine group 1 and the single medicine group 6. The specific experimental results are as shown in Table 2.

**Table 2: Influence on blood pressure of hypertension model of the single medicines and compound medicines (n=10)**

| Group | Before administration (KPa) | 1 week after administration (Kpa) | 2 weeks after administration (KPa) | 3 weeks after administration (KPa) | 4 weeks after administration (KPa) |
|---|---|---|---|---|---|
| Model group | 27.10±1.23 | 26.99±1.31 | 27.01±1.31 | 27.157±1.32 | 27.18±1.34 |
| Single medicine group 1 | 27.76±1.45 | 27.67±1.42 | 26.19±1.34^{#} | 25.41±1.18^{#} | 24.73±1.05^{#} |
| Single medicine group 2 | 27.42t1.50 | 27.28±1.48 | 25.94±1.68^{#} | 25.57±1.61^{#} | 25.29±1.49^{#} |
| Single medicine group 3 | 27.52±1.46 | 27.34±1.41 | 26.14±1.19^{#} | 25.82±1.20^{#} | 25.58±1.24^{#} |
| Single medicine group 4 | 27.45±1.07 | 27.20±1.10 | 25.99±1.39^{#} | 25.62±1.46^{#} | 25.39±1.60^{#} |
| Single medicine group 5 | 27.56±1.23 | 27.41±1.30 | 26.18±1.71^{#} | 25.83±1.92^{#} | 25.60±2.05^{#} |
| Single medicine group 6 | 27.51±1.53 | 27.30±1.56 | 26.01±1.42^{#} | 25.62±1.47^{#} | 25.42±1.45^{#} |
| Compound group 1 | 27.10±1.58 | 26.97±1.59 | 25.16±1.49^{#b1} | 24.10±1.30^{#a1b2} | 23.01±1.23^{#a2b2} |
| Compound group 2 | 26.84±1.41 | 26.67±1.44 | 24.75±1.38^{#a2b2} | 23.52+1.08^{#a2b2} | 22.27±0.96^{#a2b2} |
| Compound group 3 | 27.34±1.53 | 27.20±1.48 | 25.40±1.34^{#a1c2} | 24.35±1.24^{#a2c2} | 23.32±1.25^{#a2c2} |
| Compound group 4 | 27.03±1.43 | 26.84±1.41 | 24.95±1.30^{#2c2} | 23.78±1.25^{#a2c2} | 22.59±1.10^{#a2c2} |
| Compound group 5 | 26.83±1.60 | 26.68±1.58 | 24.99±1.49^{#} | 23.95±1.52^{#a1d2} | 22.88±1.47^{#a2d2} |
| Compound group 6 | 26.95±1.63 | 26.76±1.62 | 24.89±1.39^{#a1d2} | 23.71±1.27^{#a2d2} | 22.55±1.12^{#a2d2} |
| Compound group 7 | 27.49±1.28 | 27.32±1.22 | 25.52±1.08^{#a1e1} | 24.49±0.83^{#a2e2} | 23.50±0.84^{#a2e2} |
| Compound group 8 | 27.47±1.26 | 27.29±1.23 | 25.39±1.12^{#a2e1} | 24.23±1.02^{#a2e2} | 23.13±0.91^{#a2e2} |
| Compound group 9 | 27.30±1.58 | 27.1±1.55 | 25.42±1.33^{#a1} | 24.33±1.48^{#a2f2} | 23.35±1.39^{#a2f2} |
| Compound group 10 | 27.52±1.58 | 27.33±1.57 | 25.48±1.46^{#a2f1} | 24.35±1.35^{#a2f2} | 23.18±1.09^{#a2f2} |

| | | | | | |
|---|---|---|---|---|---|
| *Compared with the model group, p < 0.05; #compared with the model group, p < 0.01; al: compared with the single medicine group 1, p < 0.05; a2: compared with the single medicine group 1, p < 0.01; b1: compared with the single medicine group 2, p < 0.05; b2: compared with the single medicine group 2, p < 0.01, c1: compared with the single medicine group 3, p < 0.05; c2: compared with the single medicine group 3, p < 0.01; d1: compared with the single medicine group 4, p < 0.05; d2: compared with the single medicine group 4, p < 0.01; e1: compared with the single medicine group 5, p < 0.05; e2: compared with the single medicine group 5, p < 0.01; f1: compared with the single medicine group 6, p < 0.05; and f2: compared with the single medicine group 5, p < 0.01. | | | | | |

Conclusions: the compound preparations of levamlodipine and β receptor blocking agents designated in the present invention have a better anti-hypertensive effect than levamlodipine and respectively contained β receptor blocking agent, and there is significantly difference in the anti-hypertensive effects. Combined administration of levamlodipine and the β receptor blocking agent exhibits a synergistic effect on the rats having spontaneous hypertension, and has a therapeutic effect superior to those of the two medicines administrated alone.

## Claims

1. A pharmaceutical composition, comprising levamlodipine or a pharmaceutically acceptable salt thereof, and a β receptor blocking agent as active ingredient, wherein the β receptor blocking agent is one selected from nebivolol, bisoprolol, betaxolol, celiprolol, or nadolol.

2. The pharmaceutical composition according to claim 1, wherein the pharmaceutically acceptable salt of levamlodipine is selected from levamlodipine benzenesulfonate, levamlodipine mesylate, levamlodipine acetate, levamlodipine aspartate, levamlodipine tartrate, levamlodipine maleate, levamlodipine sulfate, levamlodipine hydrochloride, and levamlodipine hydrobromide.

3. The pharmaceutical composition according to claim 1, wherein in the pharmaceutical composition, a mixing ratio of levamlodipine or the pharmaceutically acceptable salt thereof to nebivolol is 1:0.2-2 by weight; a mixing ratio of levamlodipine or the pharmaceutically acceptable salt thereof to bisoprolol is 1:0.2-4 by weight; a mixing ratio of levamlodipine or the pharmaceutically acceptable salt thereof to betaxolol is 1:1-8 by weight; a mixing ratio of levamlodipine or the pharmaceutically acceptable salt thereof to celiprolol is 1:10-120 by weight; and a mixing ratio of levamlodipine or the pharmaceutically acceptable salt thereof to nadolol is 1:4-128 by weight, and in the mixing ratios, levamlodipine or the pharmaceutically acceptable salt thereof is calculated by converting into the weight of levamlodipine.

4. The pharmaceutical composition according to claim 3, wherein the mixing ratio of levamlodipine or the pharmaceutically acceptable salt thereof to nebivolol is 1:0.5-1 by weight; the mixing ratio of levamlodipine or the pharmaceutically acceptable salt thereof to bisoprolol is 1:0.5-1 by weight; the mixing ratio of levamlodipine or the pharmaceutically acceptable salt thereof to betaxolol is 1:2-4 by weight; the mixing ratio of levamlodipine or the pharmaceutically acceptable salt thereof to celiprolol is 1:20-40 by weight; the mixing ratio of levamlodipine or the pharmaceutically acceptable salt thereof to nadolol is 1:8-16 by weight, and in the mixing ratios, levamlodipine or the pharmaceutically acceptable salt thereof is calculated by being converted into levamlodipine.

5. The pharmaceutical composition according to claim 1, wherein a content of the active ingredient in per unit preparation of the pharmaceutical composition is: the content of levamlodipine or the pharmaceutically acceptable salt thereof based on levamlodipine is 1.0-30 mg; the content of nebivolol is 0.5-20 mg; the content of bisoprolol is 0.5-20 mg; the content of betaxolol is 2.5-40 mg; the content of celiprolol is 25-600 mg; and the content of nadolol is 10-640 mg.

6. The pharmaceutical composition according to claim 5, wherein the content of levamlodipine or the pharmaceutically acceptable salt thereof based on levamlodipine is 2.5-5 mg; the content of nebivolol is 1.25-5 mg; the content of bisoprolol is 1.25-10 mg; the content of betaxolol is 5-20 mg; the content of celiprolol is 50-300 mg; and the content of nadolol is 20-320 mg.

7. The pharmaceutical composition according to any one of claims 1 to 6, wherein the pharmaceutical composition further comprises a pharmaceutically acceptable adjuvant.

8. The pharmaceutical composition according to claim 7, wherein the pharmaceutically acceptable adjuvant is one or more selected from a diluent, an adhesive, a disintegrant, a lubricant, a flavoring agent, or a flavoring agent.

9. The pharmaceutical composition according to claim 7, wherein the pharmaceutical composition is an oral preparation.

10. A use of the pharmaceutical composition according to any one of claims 1 to 9 in preparation of a medicine for treating hypertension.
